Europäisches Patentamt

European Patent Office

Office européen des brevets

(11) Publication number: **0 203 403 B1**

# EUROPEAN PATENT SPECIFICATION

(45) Date of publication of patent specification: **28.10.92**

(51) Int. Cl.5: **C12N 5/08**, G01N 33/566

(21) Application number: **86105916.0**

(22) Date of filing: **29.04.86**

(54) A cloned T cell capable of recognizing tumors and a T cell antigen receptor.

(30) Priority: **01.05.85 JP 92299/85**
**08.07.85 JP 148327/85**
**10.07.85 JP 150047/85**
**05.08.85 JP 171215/85**

(43) Date of publication of application:
**03.12.86 Bulletin 86/49**

(45) Publication of the grant of the patent:
**28.10.92 Bulletin 92/44**

(84) Designated Contracting States:
**DE FR GB IT**

(56) References cited:
**EP-A- 0 149 548**

**JOURNAL OF EXPERIMENTAL MEDICINE, vol.158, November 1983, pages 1547-1560, The Rockefeller University Press; T. HERCEND et al.: "Identification of a clonally restricted 90 kD heterodimer on two human cloned natural killer cell lines"**

**NATURE, vol. 309, 28th June 1984, pages 757-762; H. SAITO et al.: "Complete primary structure of a heterodimeric T-cell receptor deduced from cDNA sequences"**

**JOURNAL OF EXPERIMENTAL MEDICINE, vol. 157, February 1983, pages 705-719, The Rockefeller University Press; S.C. MEUER et al.: "Clonotypic structures involved in antigen-specific human T cell function"**

(73) Proprietor: **Asahi Kasei Kogyo Kabushiki Kaisha**
**2-6, Dojimahama 1-chome Kita-ku**
**Osaka-shi Osaka 530(JP)**

(72) Inventor: **Kaieda, Takeji**
**168-52, Obuchi**
**Fuji-shi Shizuoka-ken(JP)**
Inventor: **Imafuku, Hiroshi**
**452-15, Ishizaka**
**Fuji-shi Shizuoka-ken(JP)**
Inventor: **Yamawaki, Naokuni**
**3-7-10, Fujimidai**
**Fuji-shi Shizuoka-ken(JP)**

(74) Representative: **Boeters, Hans Dietrich, Dr. Boeters & Bauer Bereiteranger 15 W-8000 München 90(DE)**

## Description

Heretofore, studies have been made of the identification and quantitative analysis of antigens using antibodies, inter alia monoclonal antibodies. The antibodies are antigen-recognizing substances of B lymphocytes (hereinafter often referred to as "B cells"). Especially, with respect to a tumor antigen which is produced by a tumor cell, studies have been made to obtain an antibody against a tumor antigen. Such an antibody binds to only a tumor antigen held on a tumor cell. Therefore, an antibody against a tumor cell can be utilized for clinical examination to determine whether a patient suffers from the tumor. Further, when a cytotoxin is bonded to the antibody, the cytotoxin carried by the antibody is caused to act specifically on the tumor and, as a result, the tumor is destroyed by the action of the cytotoxin. That is, the antibody can be utilized for the treatment of a tumor. In this connection, from the standpoint of clinical examination and treatment of tumors, it is desired in the art to obtain an antibody which is capable of binding to a wide variety of tumors. However, such an antibody has hitherto not been obtained.

On the other hand, according to the progress in the field of immunology, it has been found that the antigen recognition of T lymphocytes (hereinafter often referred to as "T cells") is effected by the function of a T cell antigen receptor. Therefore, it is expected that a T cell antigen receptor can also be utilized for clinic examination to determine whether a patient suffers from a tumor and for the treatment of tumor. With respect to the T cell antigen receptor, Tonegawa et al have reported the basic structure of a T cell antigen receptor[S. Tonegawa et al, Nature, 757, 309 (1984)]. According to the report of Tonegawa et al, there are about one million kinds of T cell antigen receptors. Tonegawa et al have also determined the base sequence of a DNA coding for one mouse T cell antigen receptor. Further, Mak Tak et al have determined the base sequence of a DNA coding for one human T cell antigen receptor (European Patent Application Publication No. 0 149 548). However, there has not been obtained a T cell antigen receptor which is capable of binding to a wide variety of tumors. Moreover, it is extremely difficult to obtain a sufficient amount of T cell antigen receptors, because T cells which are the sources of the T cell antigen receptors cannot be multiplied. For this reason, the study of the T cell antigen receptors has been delayed.

The present inventors have made extensive and intensive studies to cope with the above-mentioned problem and to obtain a T cell antigen receptor which is capable of binding to a wide variety of tumors. To this end, the present inventors have effected cloning of cytotoxic T cells. As a result, it has been found that there are cytotoxic T cells which are capable of reacting with and killing a wide variety of tumors but do not react with and kill normal cells. Then, the present inventors have made efforts and succeeded in obtaining clones of such cytotoxic T cells. Further, it has been found that T cell antigen receptors obtained from the clones can bind to a wide variety of tumors but do not bind to normal cells. Moreover, the present inventor have found that the T cell antigen receptors containing a cytotoxin bonded thereto can be advantageously used for killing tumor cells with no toxic effect upon normal cells, and that the T cell antigen receptors containing a marker bonded thereto can be advantageously used for the identification and quantitative analysis of tumor antigens such as cell membrane-binding tumor antigens and cell-free tumor antigens. The present invention has been made such novel findings.

Therefore, it is an object of the present invention to provide a cloned T cell which can recognize a wide variety of tumors.

It is another object of the present invention to provide a T cell antigen receptor which can bind to a wide variety of tumors.

It is a further object of the present invention to provide a marker-bonded T cell antigen receptor which can be advantageously used for clinical examination to determine whether a patient suffers from a tumor.

It is still a further object of the present invention to provide a cytotoxin-bonded T cell antigen receptor which can be advantageously used for treating tumors.

The foregoing and other objects, features and advantages of the present invention will be apparent from the following detailed description.

Essentially, according to the present invention, there is provided a cloned T cell, viz. NCACC 85 082 201 and NCACC 85 082 202, which is capable recognizing plural kinds of tumor cells.

The cloned T cell of the present invention is derived from T cells of a human since the cloned T cell of the present invention is derived from a cytotoxic T cell, the cloned T cell reacts with tumor cells and kills the tumor cells (cytotoxicty). That is, the term "recognizing tumor cells" used herein means that the cloned T cell exhibits cytotoxicity as mentioned above.

The cloned T cell of the present invention can recognize plural kinds of tumor cells. However, the cloned T cell of the present invention does not exhibit toxic activity upon normal cells. As the tumor cells, there may be mentioned, for example, solid tumors such as human gastric tumor cell lines MKN-1, MKN-7 and KATO-III, human lung tumor cell lines PC-1, PC-9, PC-10, PC-13 and PC-14, human colon tumor cell

2

lines C-1 and M7609, human rectal tumor cell lines CaR-1 and S-7512, a human cystic tumor cell line H-1, human hepatoma cell lines HLE and HLF, human bladder tumor cell lines NBT-2 and KU-1, a human throat tumor cell line KB, human kidney tumor cell lines W-2 and NRC-12, human breast tumor cell lines HBC-4 and HBC-6, a human uterine tumor cell line HeLa, human melanoma cell lines HMV-1 and HMV-2, human neuroblast tumor cell lines GOTO and SYM-I, a human ovarian tumor cell line YS-K, and a human muscular tumor cell line KYM-I. The cloned T cell of the present invention is preferably capable of recognizing at least two kinds of tumor cells. Of course, the more kinds of tumor cells the cloned T cell can recognize, the better the cloned T cell.

As specific examples of the cloned T cell of the present invention, there may be mentioned the following cloned T cells.

(1) A cloned T cell which is capable of recognizing at least two human gastric tumor cell lines MKN-1 and KATO-III but does not recognize normal human lymphocytes and fetus-derived fibroblast (this cloned T cell seems to be specific for gastric tumors).

(2) A cloned T cell which is capable of recognizing at least two human colon tumor cell lines C-1 and M7609 but does not recognize normal human lymphocytes (this cloned T cell seems to be specific for colon tumors).

(3) A cloned T cell which is capable of recognizing at least two human rectal tumor cell lines CaR-1 and S-7512 but does not recognize normal human lymphocytes (this cloned T cell seems to be specific for rectal tumors).

(4) A cloned T cell which is capable of recognizing at least two human hepatoma cell lines HLE and HLF but does not recognize normal human lymphocytes (this cloned T cell seems to be specific for hepatomas).

(5) A cloned T cell which is capable of recognizing at least two human bladder tumor cell lines NBT-2 and KU-1 but does not recognize normal human lymphocytes (this cloned T cell seems to be specific for bladder tumors).

(6) A cloned T cell which is capable of recognizing at least two human kidney tumor cell lines W-2 and NRC-12 but does not recognize normal human lymphocytes (this cloned T cell seems to be specific for kidney tumors).

(7) A cloned T cell which is capable of recognizing at least two human breast tumor cell lines HBC-4 and HBC-6 but does not recognize normal human lymphocytes (this cloned T cell seems to be specific for breast tumors).

(8) A cloned T cell which is capable of recognizing at least five human lung tumor cell lines PC-1, PC-9, PC-10, PC-13 and PC-14 but does not recognize normal human lymphocytes and fetus-derived fibroblasts (this cloned T cell seems to be specific for lung tumors).

(9) A cloned T cell which is capable of recognizing at least human lung tumor cell lines PC-10 and PC-14, human gastric tumor cell lines MKN-1 and KATO-III, and a human bladder tumor cell line NBT-2 but does not recognize normal human lymphocytes (this cloned T cell seems to have a specificity for various kinds of tumors).

Unlike natural T cells which cannot be multiplied, the cloned T cell of the present invention can be multiplied. The cloned T cell of the present invention belongs to two types of cloned T cells, i.e., a cloned T cell which may be obtained by activating a natural T cell by an activator such as lectin and interleukin 2. and a cloned T cell which is a hybridoma formed from a T cell and a T lymphoma. The former requires interleukin 2 as a growth stimulater for its growth, but the latter does not require the growth stimulater for its growth.

In the case where the cloned T cell of the present invention is a hybridoma, the T lymphoma is an 8-azaguanine resistant strain derived from human T lymphomas, viz. CEM. The cell fusion will be explained later. The above-mentioned T lymphomas are publicly available, for example, from the facilities described in Protein, Nucleic Acid, Enzyme, 23 (6), 291-304 (1978). The T lymphoma CEM is also available from the Division of Chemical Toxicology and Immunochemistry, Faculty of Pharmaceutical Science, University of Tokyo, Bunkyo-ku, Tokyo, Japan. The T lymphoma Jurkat is also available from the Department of Medicine and the Howard Hughes Medical Institute, University of California, San Francisco, California 94143, U.S.A. [Arthur Weiss & John D. Stobo, J. Exp. Med., 160, 128 (1984)]. The 8-azaguanine resistant strains may be prepared from the above-mentioned T lymphomas according to the method described in, for example, Japanese Patent Application Laid-open Specification No. 57-206383.

A cloned T cell of the invention which is capable of recognizing plural kinds of tumor cells, may be produced according to a method comprising:

(a) activating lymphocytes by an activator selected from the group consisting of a tumor cell, a lectin, interleukin 2 and mixtures thereof to obtain activated lymphocytes,

3

(b) subjecting the activated lymphocytes to cloning in a medium containing interleukin 2 to obtain cloned lymphocytes, and

(c) subjecting the cloned lymphocytes to screening to isolate a T cell capable of recognizing plural kinds of tumor cells from said cloned lymphocytes.

The lymphocytes to be used in the method of the present invention may be obtained from peripheral blood, spleen or lymph node of a mammal such as human, mouse, rat, rabbit and guinea pig. Among the peripheral blood, spleen and lymph node, the peripheral blood is more preferred because the peripheral blood can be obtained from the living body of a mammal more easily than the spleen and lymph node. The lymphocytes are activated using an activator. As the activator, there may be mentioned a tumor cell, a lectin, interleukin 2 and mixtures thereof. Of them, a lectin or interleukin 2 is more preferred because by the use of a lectin or interleukin 2, there may be obtained cloned T cells having various specificities. As the tumor cell to be used as the activator, there may be mentioned, for example, human tumor cell lines as mentioned before. As the lectin, there may be mentioned, for example, PHA derived from Phaseolus valgaris, Con A derived from Concanavalia ensiformis, WFA derived from Wisteria aoribanba, LCH derived from Lens culinaris and PWM derived from Phytolacca americana. Of them, PHA and PWM are more preferred from the standpoint of activity for activating T cells capable of recognizing tumor cells.

The activated lymphocytes are subjected to cloning. The cloning may be effected according to the method described in T. Kaieda, J. Immunol., 129, 46 (1982). Illustratively stated, the activated lymphocytes are suspended in a medium containing interleukin 2, and the thus obtained suspension is poured into each well of a microplate so that each well contains one activated lymphocyte. The microplate is then incubated to multiply each lymphocyte. Thus, cloned lymphocytes are obtained.

Then, the cloned lymphocytes are subjected to screening. The screening is effected as follows. Each cloned lymphocyte is cultured together with a tumor cell. After culturing, selection is made with respect to cloned T cells which exhibit cytotoxicity against the tumor cell. The methods for determining the cytotoxicity will be mentioned later.

Thus, there are obtained cloned T cells of the present invention which require interleukin 2 as a growth stimulater for its growth.

The thus obtained cloned T cell, i.e. NCACC 85 082 201 may, as mentioned before, be fused with a T lymphoma to form a hybridoma, i.e. NCACC 85 082 202, so that the cloned T cell may grow without a growth stimulater and can be multiplied easily.

The cell fusion of a cloned T cell and a T lymphoma may be effected according to a customary method [M. Okada, N. Yoshimura, T. Kaieda, Proc. Natl. Acad. Sci., 78, 7717 (1981)]. Illustratively stated, the cell fusion may be carried out in a customary medium containing a fusion accelerator. As the fusion accelerator, there may be mentioned, for example, polyethylene glycol (hereinafter often referred to as "PEG"), etc. The amount ratio of the cells of a T lymphoma to the cells of the cloned T cell may generally be about 1 to 10. The cells of the cloned T cell and T lymphoma are mixed in the medium at room temperature. The resulting mixture is subjected to centrifugation to separate the mixture into cells and a supernatant. The supernatant is discarded. To the cells is added a medium containing PEG which has been heated at 37 °C, followed by stirring to initiate the cell fusion. The mixture is incubated to advance the fusion reaction. During the incubation, the medium is changed to a fresh medium, that is, the mixture is subjected to centrifugation to separate the mixture into cells and a supernatant, and then the supernatant is discarded, and to the cells is added a medium. The change of the medium is effected at predetermined intervals. Thus, a hybridoma is formed.

The hybridoma is isolated from the cells remaining unfused. The isolation is effected by culturing the cell mixture containing the hybridomas and the cells remaining unfused in a customary medium for hybridoma selection. The medium for hybridoma selection is a medium in which the hybridoma can grow but the unfused cells cannot grow. As such a medium, there may be mentioned, for example, a medium containing hypoxanthine, aminopterine and thymidine (hereinafter referred to as "HAT medium"). As the HAT medium, there may be mentioned, for example, a medium containing 10 % fetal calf serum (FCS), 2 x $10^{-7}$ M aminopterine, 1 x $10^{-4}$ M hypoxanthine, 1.6 x $10^{-3}$ M thymidine and 3 x $10^{-6}$ M glycine. Culturing of the cells in a HAT medium is effected according to a customary limiting dilution method for a sufficient period of time so that cells other than the hybridoma die. Thus, the intended hybridoma can be selectively obtained.

The thus obtained hybridoma is different from the original 8-azaguanine resistant T lymphoma and the original cloned T cell with respect to, for example, karyotype (number of chromosomes), mitogen response, lymphokine producibility. The hybridoma may be multiplied and stored in a HAT medium as mentioned above. However, it is preferred that after the selection of the hybridoma, the hybridoma be cultured in a customary HT medium containing hypoxanthine and thymidine and, then, the hybridoma be transferred into a customary growth medium.

The hybridoma of the present invention may also be prepared by a method in which lymphocytes are fused with T lymphomas to obtain hybridomas and, then, the hybridomas are subjected to screening to obtain a hybridodma capable of recognizing tumor cells. That is, in even another aspect of the present invention, there is provided a method for producing a cloned T cell which is capable of recognizing plural kinds of tumor cells, comprising:

(a) fusing lymphocytes with cells of a T lymphoma to form hybridomas, and

(b) subjecting the hybridomas to screening to isolate a T cell capable of recognizing plural kinds of tumor cells from said hybridomas.

As the lymphocytes to be fused with cells of a T lymphoma, there may be employed the lymphocytes as mentioned before. As the T lymphoma, there may be employed 8-azaguanine resistant strains as mentioned before. The cell fusion may be effected in the same manner as mentioned before. Thus, there are obtained a hybridoma mixture including hybridomas which can recognize tumor cells and hybridomas which cannot recognize tumor cells. From the hybridoma mixture, the hybridomas capable of recognizing tumor cells are isolated by the screening method as mentioned before.

Thus, a cloned T cell of the present invention is obtained in the form of a hybridoma.

In a further aspect of the present invention, there is provided an antigen receptor of a T cell, viz. NCACC 85 082 201 and NCALL 85 082 202, which is capable of binding to plural kinds of tumor cells.

The T cell antigen receptor of the present invention is bonded to the surface of the T cell. The T cell antigen receptor of the present invention is obtained from the above-mentioned cloned T cell of the present invention. The method for producing a T cell antigen receptor from the cloned T cell will be explained below.

First, the cloned T cells are cultured in a customary medium or in the body of an X ray-irradiated animal on a large scale. The cloned T cells are collected. From the thus collected cells, a T cell antigen receptor is efficiently obtained from the culture according to a customary method [see, for example, S. C. Meuer, J. Exp. Med., 157, 705 (1983)]. Illustratively stated, the cloned T cells are suspended in a PBS (a phosphate buffer containing 0.85 % NaCl, pH7.2) containing 1 % Triton X-100. The resulting suspension is stirred on ice for 1 hour so that membrane proteins of the cloned T cell are dissolved in the suspension. Then, the suspension is subjected to affinity column chromatography using a column packed with Sepharose 4B (manufactured and sold by Pharmacia Fine Chemicals AB, Sweden) to which a monoclonal antibody against a T cell antigen receptor is bonded. The monoclonal antibody against a T cell antigen receptor may be prepared by a method as described in the later-mentioned Example 3.

Thus, there is obtained a T cell antigen receptor in substantially pure form.

The thus obtained T cell antigen receptor has a molecular weight of about 90,000 in terms of a value as measured by SDS-polyacylamide gel electrophoresis under a unreduced, condition. However, when the T cell antigen receptor is subjected to SDS-polyacrylamide gel electrophoresis under a reduced condition, there are found two bands on the gel, i.e., a band corresponding to a molecular weight of about 50,000 and a band corresponding to a molecular weight of about 45,000. This shows that the T cell antigen receptor is a heterodimer comprising a protein having a molecular weight of about 50,000 and, covalently bonded thereto through a disulfide bond, a protein having a molecular weight of about 45,000.

On the other hand, when the T cell antigen receptor is obtained from the cloned T cell of the present invention after the cloned T cell is treated with tunicamycin, the thus obtained T cell antigen receptor has a molecular weight of about 60,000 in terms of a value as measured by SDS-polyacrylamide gel electrophoresis under a non-reduction condition. Further, when the thus obtained T cell antigen receptor is subjected to SDS-polyacrylamide gel electrophoresis under a reduction condition, it is found that the T cell antigen receptor is a heterodimer comprising two proteins each having a molecular weight of about 30,000. These results show that the T cell antigen receptor of the present invention is a heterodimer comprising two proteins each containing sugar. The above-mentioned molecular weights of the T cell antigen receptor are consistent with those of a T cell antigen receptor reported in J. Exp. Med., 158, 1547 (1983). The T cell antigen receptor of the present invention may or may not contain sugar.

The purity of the T cell antigen receptor of the present invention is about 95 % or more. The purity may be determined by subjecting the T cell antigen receptor to SDS-polyacrylamide gel electrophoresis, followed by densitometry using a densitometer model ADC-20EX (manufactured and sold by Kayagaki Co., Ltd., Japan).

The T cell antigen receptor of the present invention may also be produced by a customary recombinant DNA technique. Illustratively stated, mRNAs are obtained from a cloned T cell of the present invention which contain an intended T cell antigen receptor, and using the thus obtained mRNAs, cDNAs are synthesized. From the thus synthesized cDNA, a cDNA coding for the intended T cell antigen receptor is isolated by means of cloning. The thus isolated cDNA is ligated to an appropriate expression vector to obtain a recombinant DNA. With the recombinant DNA, cells of E. coli, a yeast or an animal are transfected to obtain a transformant. The transformant is cultured in an appropriate medium, causing the transformant to produce the T cell antigen receptor.

The T cell antigen receptor of the present invention can bind to plural kinds of tumor cells as mentioned above with respect to the cloned T cell. The T cell antigen receptor is preferably capable of binding to at least two kinds of tumor cells. Of course, the more kinds of tumor cells to which the T cell antigen receptor can bind, the better the T cell antigen receptor.

In still a further aspect of the present invention, there is provided an cell/antigen receptor of a T cell, viz. NCACC 85 082 201 and NCACC 85 082 202 which is capable of binding to plural kinds of tumor cells and contains a marker bonded thereto.

The marker-bonded T cell antigen receptor of the present invention may be obtained by bonding a marker to the T cell antigen receptor as mentioned before. As the marker, there may be mentioned, for example, fluorescent substances such as fluorescein isothio-cyanate (FTC) and rhodamine, enzymes such as peroxidase, $\beta$-galactosidase and glucose oxidase, and radio-isotopes such as $^{125}$I, $^{131}$I, $^{32}$S, $^{14}$C and $^{3}$H.

The marker may be bonded to a T cell antigen receptor according to a customary method for obtaining a labeled antibody. As such a method, there may be mentioned, for example, a method in which FITC is bonded to a T cell antigen receptor under an alkaline condition, a method in which a maleimide group is introduced into an enzyme, and the resulting enzyme is bonded to a T cell antigen receptor through N-succinimidyl 3-(pyridyldithio)propionate (SPDP), a method in which an enzyme is bonded to an SH group of a T cell antigen receptor, and a method in which a T cell antigen receptor is labeled with $^{125}$I by a customary lactoperoxidase method,. However, the method for bonding a marker to a T cell antigen receptor varies depending upon the kind of the marker and, therefore, the method should not be limited to the above-mentioned methods.

The amount of the marker bonded to the T cell antigen receptor varies depending upon the kind of the marker. Generally, the marker may be bonded to the T cell antigen receptor in an amount of about 1 to about 10 moles/mole of the T cell antigen receptor.

In still a further aspect of the present invention, there is provided an antigen receptor of a T cell, viz. NCACC 85 082 201 and NCACC 85 082 202, which is capable of binding to plural kinds of tumor cells and contains a cytotoxin bonded thereto.

The cytotoxin-bonded T cell antigen receptor of the present invention may be obtained by bonding a cytotoxin to the T cell antigen receptor as mentioned before. As the cytotoxin, there may be mentioned, for example, anticancer drugs, biological toxic substances derived, for example from plants or animals, light-activated toxins and radioisotopes. As the anti-cancer drugs, there may be mentioned, for example, alkyl reagents such as cyclophosphamide and nitrogen mustard, metabolic antagonists such as $N^4$-behenoil ara-C, fluorouracil, 1-franocyl-5-fluorouracil and mercaptopurin, antibotics such as bleomycin, mitomycin, actnomycin D and cycloheximide, and hormones such as prednisone and prostaglandin. As the biological toxic substances, there may be mentioned, for example, toxins such as snake toxin, diphtheria toxin and ricin. As the light-activated toxin, there may be mentioned, for example, hematoporphyrin derivatives. As the radioisotopes, there may be mentioned, for example, $^{125}$I, $^{60}$Co, $^{90}$Sr, $^{32}$P and $^{192}$Ir.

The cytotoxin may be bonded to a T cell antigen receptor by a customary method. As the method for bonding a cytotoxin to a T cell antigen receptor, there may be mentioned, for example, a method in which the ricin A chain of toxin of a bean is bonded to a T cell antigen receptor through N-succinimidyl 3-(2-pyridyldithio) propionate (SPDP) to form a complex, a method in which a T cell antigen receptor is bonded to a surface of a ribosome containing an anticancer agent such as actinomycin D, a method in which an anticancer agent such as adriamycin is bonded to a T cell antigen receptor using a dextran as a crosslinking agent to forms, for example, a complex. However, the method for bonding a cytotoxin to a T cell antigen receptor varies depending upon the kinds of the cytotoxin and, therefore, the method should not be limited to the above-mentioned methods.

The amount of the cytotoxin bonded to the T cell antigen receptor varies depending upon the kind of the cytotoxin. Generally, the cytotoxin may be bonded to the T cell antigen receptor in an amount of about 1 to about 100 moles/mole of the T cell antigen receptor.

The cloned T cell of the present invention can recognize plural kinds of tumors due to the T cell antigen receptor held on the cloned T cell, which receptor can bind to plural kinds of tumors. The cloned T cell can be multiplied, as is different from natural T cells. Therefore, the cloned T cell of the present invention is useful as a raw material for obtaining a T cell antigen receptor of the present invention. Especially, it is advantageous when the cloned T cell is a hybridoma, because the cloned T cell can be multiplied more easily than the cloned T cell which is not a hybridoma. The T cell antigen receptor of the present invention can specifically bind to plural kinds of tumors but does not bind to normal cells. Therefore, the T cell antigen receptor can be advantageously employed for clinical examination to determine whether a patient suffers from a tumor and for treatment of a tumor. Illustratively stated, when a marker is bonded to the T cell antigen receptor, the T cell antigen receptor can be advantageously used for identification and quantitative analysis of tumor antigen on a tumor cell membrane and cell-free tumor antigen. On the other hand, when a cytotoxin is bonded to the T cell antigen receptor, the T cell antigen receptor can be advantageously used for treatment of tumors such as gastric tumor, lung cancer and breast tumor. The T cell antigen receptor of the present invention can also bind to antigens such as virus antigen, bacterial antigen and MHC antigen. Therefore, the T cell antigen receptor can also be advantageously employed for the identification and quantitative analysis of such antigens and for the removal of malignant cells such as virus-infected cells and pathogenic cells in vivo.

In practicing the clinical examination using the marker-bonded T cell antigen receptor of the present invention, the amount of the marker-bonded T cell antigen receptor to be employed varies upon the kind of the marker. Generally, the marker-bonded T cell antigen receptor of the present invention may be employed in an amount of about 1 ng to 10 $\mu$g per sample (such as serum, tissues, etc.) to be examined.

In practicing the treatment of tumors using the cytotoxin-bonded T cell antigen receptor of the present invention, the amount of the cytotoxin-bonded T cell antigen receptor to be administered varies depending upon the body weight, age, sex, etc. of a patient to be treated. Generally, the cytotoxin-bonded T cell antigen receptor may be administered to a patient in an amount of about 10 $\mu$g to about 1 mg/kg of the body weight of a patient.

The present invention will now be described in detail by reference to the following Examples, which should not be construed to limit the scope of the present invention.

In the following Examples, tumor cells used for screening are publicly available from the facilities described in Protein, Nucleic Acid, Enzyme 23, 697 (1978), published by Kyoritsu Shuppan, Tokyo.

Of the cloned T cells and hybridomas, respectively of the present invention obtained in the following Examples, the following cloned T cells have been deposited at Public Health Laboratory Service Center for Applied Microbiology Research (Porton Down, Salisbury Wiltshire, SP40JG, U.K.)

| Cloned T cell | Deposit No. | |
|---|---|---|
| Human cloned T cell | clone 51 | 85082201 |
| Human hybridoma | H51-4-D1 | 85082202 |

Example 1

(Establishment of human cloned cytotoxic T cells which are capable of killing tumors)

The cloning of human T cells and screening of the cloned cytotoxic T cells capable of recognizing tumors were effected as follows.

First, human lymphocytes were obtained as follows. Human peripheral blood was collected from an adult man, diluted two-fold with Hank's solution (manufactured and sold by Nissui Pharmaceutical Co., Ltd., Japan), overlaid on Ficoll-Paque solution (manufactured and sold by Pharmacia Fine Chemicals AB, Sweden) and subjected to centrifugation at 2000 rpm for 20 min. Thus, there was obtained a mixture in the form of layers. The middle layer which is the lymphocyte layer was collected from the mixture, washed with Hank's solution, and then suspended at a concentration of 2 x $10^6$ cells/m$\ell$ in RPMI 1640 medium (manufactured and sold by Nissui Pharmaceutical Co., Ltd., Japan) to which fetal bovine serum had been added in an amount of 10 (w/v) %. To the suspension was added a lectin PHA-P (Difco Laboratores, U.S.A.) to a concentration of 0.1 %, and the mixture was placed in a culture bottle and cultured at 37 °C for 48 hours in air containing 5 % $CO_2$. Thus, the lymphocytes were activated by lectin (PHA-P), and various cytotoxic T cells and helper T cells which can recognize various human tumor cell lines were induced. In order to obtain a cloned cell of each activated lymphocyte, the activated lymphocytes were subjected to cloning according to the method of Kaieda [T. Kaieda, Meneki Jikken Sosa-ho (Experimental methods in Immunology) XI, p.689, edited by Japanese Society of Immunology]. Illustratively stated, the activated lymphocytes were suspended at the concentration of 5 cells/m$\ell$ in RPMI 1640 medium containing 20 v/v% fetal bovine serum and interleukin 2 (manufactured and sold by Boehringer-Mannheim Co., Ltd., West Germany). To the resulting suspension were added self-lymphocytes treated with a solution containing 100 $\mu$g/m$\ell$ of Mitomycin-C (manufactured and sold by Kyowa Hakko Kogyo Co., Ltd., Japan) at 37 °C for 45 min so that at the self-lymphocyte concentration became $1 \times 10^5$ cells/ml. The resulting mixture was put into each well of 96 well plate (Falcon No. 3072 manufactured and sold by Falcon, U.S.A.) in an amount of 200 μl and cultured in air containing 5 % $CO_2$ at 37 °C for 2 weeks to form cloned T cells. Whether these cloned T cells recognize tumor cells was determined through evaluation of the cytotoxicity of the cloned T cells against tumor cells. The evaluation of the cytotoxicity is as follows. First, various tumor cells were labeled using $^{51}Cr$ according to a customary method [R.M. Thorn et al, J. Immunol Methods 4, 301 (1974)], and $1 \times 10^4$ cells of the labeled tumor cell and $1 \times 10^5$ cells of each cloned T cell were mixed and cultured in 200 μl of RPMI medium containing 5 v/v% fetal bovine serum (in air containing 5 % $CO_2$ at 37 °C). Then, by counting the radioactivity of $^{51}Cr$ released in the supernatant of the cultured mixture, the cytotoxicity of the cloned T cells was evaluated.

The cytotoxicity was calculated according to the following formula.

Cytotoxicity (%) = [(B-C)/(A-C)] x 100

(wherein A is the radioactivity of $1 \times 10^4$ cells of the $^{51}Cr$-labeled tumor cell, B is the radioactivity in the supernatant of the cultured mixture in which $1 \times 10^4$ cells of the $^{51}Cr$-labeled tumor cells and $1 \times 10^5$ cells of the cloned T cell were cultured, and C is the radioactivity in the supernatant of a medium in which only $1 \times 10^4$ cells of $^{51}Cr$-labeled tumor cell were cultured.)

The cloned T cell of which the cytotoxicity against a tumor cell was 10 % or more was regarded as being capable of recognizing the tumor cell.

10,000 cells were subjected to cloning under the conditions as described above. As a result, about 500 kinds of cloned T cells were obtained. Of them, about 100 kinds of the cells exhibited cytotoxicity against at least one kind of the human tumor cell lines (cloned human cytotoxic T cells). The above-mentioned cloning procedures were repeated to obtain 21 cloned human cytotoxic T cells capable of killing at least two kinds of tumor cell lines (Nos. 4, 8, 15, 19, 24, 28, 36, 49, 51, 75, 83, 90, 95, 115, 131, 138, 150, 165, 172, 188 and 5B5). The cytotoxicities of the thus obtained clones against various tumor cells and fibroblasts derived from a fetus are shown in Tables 1 and 2. These clones required interleukin 2 for their growth. Therefore, the clones were cultured in RPMI 1640 medium containing 20 v/v% fetal bovine serum and interleukin 2. Every 7 to 10 days, the PHA-P and human self peripheral blood lymphocytes from which the clones were derived were treated with mitomycin and added to the culture of the clones. The clones were capable of multiplying even after the clones were cultured for 6 months. Through the repetition of the above-mentioned procedures, it was affirmed that the cloned human T cells were obtained with high reproducibility.

### Table 1

Cytotoxicity of cloned human cytotoxic T cells against various tumor cells

| | | | Cytotoxicity of cloned human cytotoxic T cells | | | | | | | | | | |
|---|---|---|---|---|---|---|---|---|---|---|---|---|---|
| | | | clones | | | | | | | | | | |
| | | | 4 | 8 | 15 | 19 | 24 | 28 | 36 | 49 | 51 | 75 | 83 |
| human tumor cell lines | gastric tumor | MKN-1 | | | | | | | | | + | + | + |
| | | KATOIII | − | + | − | − | − | − | − | − | + | + | + |
| | | PC-1 | | | | + | | | | | − | | |
| | lung tumor | PC-9 | − | − | − | − | − | − | − | − | − | + | + |
| | | PC-10 | | | | | + | | | | + | + | + |
| | | PC-13 | − | − | − | + | − | − | − | − | + | + | |
| | | PC-14 | | | | + | | | | | + | + | + |
| | colon tumor | C-1 | + | − | − | − | − | − | − | − | − | + | |
| | | M7609 | + | | | | | | | | | | |
| | rectal tumor | CaR-1 | − | − | + | − | − | − | − | − | | | |
| | | S-7512 | | | + | | | | | | | | |
| | cystic tumor | H-1 | | | | | | | | − | | + | + |
| | hepatoma | HLE | − | − | − | − | + | − | − | − | − | | |
| | | HLF | | | | | + | | | | | | |
| | bladder tumor | NBT-2 | | | | | | | | + | + | + | + |
| | | KU-1 | | | | | | | | + | | | |
| | throat tumor | KB | | | | | | | | | + | + | + |
| | kidney tumor | W-2 | − | − | − | − | − | + | − | − | − | + | |
| | | NRC-12 | | | | | | + | | | | | |
| | breast tumor | HBC-4 | − | − | − | − | − | − | + | − | | | |
| | | HBC-6 | | | | | | | + | | | | |
| | uterine tumor | HeLa | | | | | | | | | + | + | + |
| | melanoma | HMV-1 | − | − | − | − | − | − | − | − | + | + | + |
| | | HMV-2 | | | | | | | | | + | | |
| fetal cells | tongue | HET | | | | | − | | | | + | + | − |
| | lung | HEL | | − | | | | | | | − | − | − |
| | foreskin | MRHF | | | | | | | | | − | − | − |
| normal lymphocytes | | | − | − | − | − | − | − | − | − | − | − | − |

cytotoxicity: − (less than 10%), + (10% or more)

## Table 2

### Cytotoxicity of cloned human cytotoxic T cells against various target cells

Cytotoxicity of
cloned human cytotoxic T cells

|  |  | clones | | | | | | | | | |
|---|---|---|---|---|---|---|---|---|---|---|---|
|  |  | 90 | 95 | 115 | 131 | 138 | 150 | 165 | 172 | 188 | 5B5 |
| gastric tumor | MKN-1 | + | + | - | - | - | - | - | + | - | + |
|  | KATO-III | + | - | - | - | + | - | - | + | - |  |
|  | PC-9 | - | - | - | - | - | - | - | + | - |  |
| lung tumor | PC-10 | - | - | + | - | - | - | - | + | - | + |
|  | PC-13 |  |  | + |  |  |  |  |  |  | + |
|  | PC-14 |  |  | + |  |  |  |  |  |  |  |
| colon tumor | C-1 | + | + | - | - | - | + | - | + | - |  |
| cystic tumor | H-1 | + | - | - | - | - | - | - | + | - |  |
| bladder tumor | NBT-2 | + | - | - | + | - | - | + | + | + | + |
| throat tumor | KB | - | + | - | + | + | + | - | + | - | + |
| kidney tumor | W-2 |  |  |  |  |  |  | + | + | + |  |
| uterine tumor | HeLa | - | - | - | + | + | + | + | + | - |  |
| melanoma | HMV-1 | - | - | - | - | - | - | - | + | - |  |
| normal lymphocytes |  | - | - | - | - | - | - | - | - | - | - |

cytotoxicity:  - (less than 10%),  + (10% or more)

Example 2

(Preparation of a hybridoma derived from the clone 51)

The cell line CEM-AG[R] was used as the 8-azaguanine resistant human T-lymphoma. The cell line CEM-AG[R] was prepared according to the method described in Japanese Patent Application Laid-Open Specification No. 57-206383. The cells of the cell line were suspended in an RPMI 1640 medium containing 20 v/v % FCS and 100 $\mu$M of 8-azaguanine. The resulting suspension was cultured for 2 days while the medium was changed to a fresh medium every day by the method in which the culture was subjected to centrifugation to separate the culture into cells and a supernatant, and after the supernatant was discarded, to the cells was added a fresh medium. The cells multiplied actively. Then the cells were collected by centrifugation. The collected cells were suspended in an RPMI 1640 medium containing 20 v/v % FCS and allowed to stand for one day. 6 x 10[7] cells of the above-obtained cell line CEM-AG[R] and 8 x 10[5] cells of the human cytotoxic T cell 51 obtained in Example 1 which was capable of recognizing a wide variety of tumor cells were subjected to cell fusion. That is, both the above-mentioned cells were washed three times with a MEM medium not containing FCS which had been preheated to 37 ° C, and suspended in the MEM medium. Then, the cell suspension was put in a 50 mℓ conical tube, mixed sufficiently and centrifuged at 800 rpm at room temperature for 10 min to separate the suspension into a cell pellet and a supernatant. After removing the supernatant, the resulting cell pellet was shaken gently. To the cell pellet was added 0.5 mℓ of a MEM solution containing 45 w/v % of PEG-6000 (manufactured and sold by Kochlight, England), which solution had been heated to 37 °C. The resulting mixture was shaken sufficiently for 30 seconds, and allowed to stand in a $CO_2$ gas incubator filled with a gas containing 5 % $CO_2$ and 95 % air at 37 °C for 6 min. Then, 12 mℓ of a MEM medium (preheated at 37 °C) not containing FCS was added to the mixture at a rate of 2 mℓ per minute while the conical tube was rotated. Then, 25 mℓ of a MEM medium was rapidly added to the mixture. The mixture was centrifuged at 800 rpm at room temperature for 10 min to separate the mixture into a cell pellet and a supernatant, and the supernatant was removed. The obtained pellet was loosened. Then, 120 mℓ of an RPMI 1640 medium containing 30 v/v% FCS (preheated to 37 °C) was gently added to the loosened pellet to give a suspension having a CEM-AG$^R$ cell concentration of 5 x 10$^5$ cells/mℓ. 1 mℓ of the thus obtained suspension was put in each of 120 wells of five 24 well culture plates (Costar No. 3524, manufactured and sold by Costar, U.S.A.). 24 hours later, 1 mℓ of HAT medium [an RPMI 1640 medium containing 1 x 10$^{-4}$ M hypoxanthine (manufactured and sold by Sigma Chemical Company, U.S.A.), 2 x 10$^{-7}$ M aminopterine (manufactured and sold by Sigma Chemical Company, U.S.A.), 1.6 x 10$^{-5}$ M thymidine (manufactured and sold by Sigma Chemical Company, U.S.A.) and 20 v/v % FCS] was added to each well. Half of the supernatant of the culture medium in each well was removed and 1 mℓ of a fresh HAT medium was added to each well every two days and the culturing was effected in a 5 % $CO_2$ gas incubator at 37 °C for 3 weeks. The cell lines thus multiplied here then put in a HT medium (the same medium as the HAT medium except that aminopterine is not present and further cultured for 1 week. The cell lines were then transferred into a normal medium (an RPMI 1640 medium containing 20 v/v % FCS but not containing HAT). As a result, 6 kinds of hybridomas were obtained. The hybridomas thus obtained were subjected to assay to determine whether or not the obtained hybridomas had produced T cell antigen recepters. The assay was effected by staining the hybridomas according to the so-called indirect method using anti-T cell antigen recepters as a first antibody and FITC labeled antimurine Ig antibody (manufactured and sold by TAGO Inc., U.S.A.) as a second antibody. The thus stained hybridomas were examined by fluorescence microscopy. As a result, it was found that among the 6 kinds of clones of hybridomas, only 1 kind of clone had a ring-shaped stained portion. The results show that only one kind of clone had produced a T cell antigen receptor. This clone was named H51-4. On the other hand, the parent cell line CEM-AG$^R$ was not stained at all by this assay method. The clone H51-4 contained both of cells which had a clearly stained portion, that is, had a large amount of a T cell antigen receptor, and cells which were stained a little, that is, had a small amount of a T cell antigen receptor. Therefore, in order to obtain a hybridoma capable of producing a large amount of a T cell antigen receptor, subclones of the clone H51-4 were obtained by effecting cloning according to a customary method as follows. First, 1 x 10$^7$ cells of the cell line CEM-AG$^R$ were washed with a customary growth medium and suspended in 60 mℓ of the HAT medium. Then, both the cells of the clone H51-4 were suspended in the medium at a concentration of 2.5 cells/mℓ, and added to the above-mentioned HAT medium. 0.2 mℓ of the resulting mixture was put in each well of a 96 well microplate having a capacity of 200 μl/well (Falcon No. 3072, manufactured and sold by Falcon, U.S.A.). Every two weeks, half of the supernatant of the mixture in each well was removed and a fresh HAT medium preheated to 37 °C was added to each well to keep the above-mentioned cells multiplying. Thus, there were obtained subclones of the clone H51-4. The cells of the thus obtained subclones were subjected to staining by the above-mentioned indirect method to select from the above-obtained subclones, the subclones of which almost all the cells had a clearly stained portion. As a result, there were selected two subclones. The thus selected subclones produced a T cell antigen receptor in large amount very stably. The selected subclones were named H51-4-A2 and H51-4-D1.

Example 3

(Culturing of cloned human cytotoxic T cells, $^{131}$I labeling of membrane proteins of the cells and preparation of soluble membrane protein fraction of the cells)

The human cytotoxic T cell clone 51 obtained in Example 1 was cultured in the presence of interleukin 2 to obtain 2 x 10$^9$ cells. From the cells, T cell antigen receptors were obtained as follows. The cells were washed three times with Hank's solution, suspended in 2 mℓ of PBS (containing 1 mM phenyl methyl sulfonyl fluoride, 1 mM EDTA and 10 mM NaF; pH 7.2) containing 1 % Triton X-100 and stirred in ice for 1 hour so that the membrane proteins of the cells were made soluble. The mixture was subjected to centrifugation at 10000 g for 20 min to remove the debris of the cells from the mixture. Thus, there was obtained a soluble membrane protein fraction.

(Preparation of anti-T cell antigen receptor monoclonal antibody)

An anti-T cell antigen receptor monoclonal antibody was obtained as follows. First, $1 \times 10^8$ cells of human peripheral blood lymphocytes were suspended in 100 mℓ of an RPMI medium (containing 0.1 % PHA-P) containing 10 v/v% fetal bovine serum and cultured for two days. Then, the multiplication of the cells was effected in a medium containing interleukin 2 to obtain $1 \times 10^9$ cells of activated T cells. The obtained cells were treated in substantially the same manner as mentioned before to obtain a soluble membrane protein fraction of the cells. The fraction was then placed on Sephacryl S-200 (manufactured and sold by Pharmacia Fine Chemicals AB, Sweden) and subjected to gel chromatography using PBS as an eluent to collect fractions having molecular weights of 70,000 to 100,000, respectively. The fractions were put together and concentrated to a volume of 0.5 mℓ. The thus obtained concentrate was mixed with an equivolume of Freund's complete adjuvant. The resulting mixture was injected to a BALB/c mouse intraperitoneally. 10 days later and 20 days later, 100 μℓ of the above-obtained concentrate was twice injected to the mouse intraperitoneally. 10 days after the last injection of the concentrate, 100 μℓ of the concentrate was injected to the mouse intravenously to complete the immunization of the mouse. 3 days after the intravenous injection, the spleen cells of the immunized mouse were taken out and fused with murine myeloma P3UI by a customary method [G. Kohler & C. Milstein; Nature 256, 49 (1975)], thereby to obtain hybridomas. The hybridomas were subjected to screening with respect to the binding activity, to human T cells, of a supernatant obtained from the culture of the hybridoma and with respect to the presence of antibody in the supernatant, which antibody was able to bind to a peptide of human T cell antigen receptor $\beta$ constant region which had been organo-chemically synthesized according to a customary solid phase method based on the amino acid sequence of a T cell antigen receptor described in Nature, 308, 145 (1984). Thus, there was obtained a hybridoma producing anti-T cell antigen receptor antibody. The supernatant of the hybridoma was subjected to immunoprecipitation with the T cell membrane protein fraction as obtained before and subjected to SDS-polyacrylamide gel electrophoresis. As a result, the precipitates were formed and there were observed a band corresponding to the molecular weight of 90,000 under an unreduced condition, and two bands corresponding to molecular weights of 50,000 and 45,000 under a reduced condition. Thus, it was confirmed that the obtained hybridoma produced anti-T cell antigen receptor monoclonal antibody. The hybridoma was named 116-23.

(Preparation of anti-T cell antigen receptor monoclonal antibody-bonded Sepharose 4B)

The hybridodma (116-23) was cultured in 1 ℓ of an RPMI medium containing 20 v/v% fetal bovine serum. The culture was subjected to purification by a customary method by means of affinity chromatography using a Protein A-agarose column [T. Iwasaki, "Monoclonal antibody", published by Kodansha, Tokyo, p 175-177 (1983)], thereby to obtain 10 mg of anti-T cell antigen receptor monoclonal antibody. 1 mg of the obtained antibody was bonded to 2 g of CNBr-activated Sepharose 4B (manufactured and sold by Pharmacia Fine Chemicals AB, Sweden) as follows. First, 1 g of CNBr-activated Sepharose 4B was sufficiently washed with 1 mM (200 mℓ) HCl on a glass filter (G3), further washed with 200 mℓ of a coupling buffer (0.1 M $NaHCO_3$-$Na_2CO_3$ buffer, pH 8.3, 0.5 M NaCl) and dried. Then, the antibody was dissolved in the coupling buffer in such an amount that the resulting solution had the antibody concentration of 1 mg/mℓ. To 4 mℓ of the thus obtained solution was added the dried Sepharose, and the mixture was reacted at room temperature for 2 hours while gently stirring. 2 hours later, 5 mℓ of 0.2 M glycine (pH 8.0) was added to the reaction mixture, and the resulting mixture was reacted at room temperature for 2 hours to block excess active groups. The obtained mixture was placed on a glass filter (G3) and washed successively with 200 mℓ of the coupling buffer, 200 mℓ of an acetate buffer (0.1 M sodium acetate, pH 4, 0.5 M NaCl) and 200 mℓ of a phosphate buffer (pH 7.0, 0.15 M NaCl) to obtain antibody-bonded Sepharose.

(Purification of T cell antigen receptor by affinity chromatography)

The above-obtained soluble membrane protein fraction of the human cytotoxic T cell clone 51 was put into a column packed with 3 mℓ of anti-T cell antigen receptor monoclonal antibody-bonded Sepharose 4B to cause the T cell antigen receptor to be adsorbed. Then, 200 mℓ of the PBS was flowed through the column to elute impurities. Subsequently, 10 mℓ of 0.1 M glycine-hydrochloric acid (pH 3.0) was added to the column to elute the adsorbed T cell antigen receptor. The eluate was neutralized and then concentrated to obtain 0.5 mℓ of a concentrate. The concentrate was subjected to SDS-polyacrylamide electrophoresis. As a result, there were detected a band corresponding to the molecular weight of about 90,000 under an unreduced condition. Further, there were detected two bands corresponding to the molecular weights of about 50,000 and about 45,000 under a reduced condition. Thus, it was affirmed that the clone 51 produced T cell antigen receptor.

(labeling with FITC of T cell antigen receptor)

The above-obtained concentrate containing 1 $\mu$g of the T cell antigen receptor was subjected to dialysis against a phosphate buffer (10 mM sodium phosphate, 0.85 % NaCl, pH 7.2) to obtain 1 m$\ell$ of the T cell antigen receptor solution. 1 m$\ell$ of the T cell antigen receptor solution, 0.1 m$\ell$ of 0.5 M carbonic acid-bicarbonate buffer (pH 9.3) and 10 $\mu\ell$ of 4 $\mu$g/m$\ell$ FITC solution (manufactured and sold by Sigma Chemical Company, U.S.A.) were put in a small beaker and reacted at room temperature for 6 hours. After the reaction, the mixture was dialyzed against a phosphate buffer, and 1 m$\ell$ of a dialysate was stored at -20 °C.

(Binding activity of FITC-labeled T cell antigen receptor to tumor cells)

0.1 m$\ell$ of the thus obtained FITC-labeled T cell antigen receptor derived from the clone 51 was mixed with an RPMI 1640 medium containing 5 v/v% fetal bovine serum and, suspended therein, various tumor cell lines recognized by the clone 51 (MKN-1, KATO-III, PC-10, NBT-2) at a concentration of 1 x $10^6$ cells/m$\ell$. The mixture was kept at 0 °C for 1 hour. Then, the mixture was washed with Hank's solution and observed under a fluorescence microscope. As a result, it was observed that these tumor cell lines emitted fluorescence of a ring shape. On the other hand, the cells which were not recognized by the clone 51 did not emit fluorescence. Thus, it was affirmed that the T cell antigen receptor acted as a tumor cell recognizing substance.

Example 4

A T cell antigen receptor was obtained from the clone 51 derived hybridoma H51-4-D1 obtained in Example 2 in substantially the same manner as in Example 3 and the binding activity thereof to tumor cells was investigated. As a result, it was found that the receptor bound to MKN-1, KATO-III, PC-10 and NBT-2, but not to the tumor cells which were not recognized by the clone 51.

Example 5

A T cell antigen receptor was prepared from the clone 51 in substantially the same manner as in Example 1. The T cell antigen receptor was $^{125}$I-labeledby the lactoperoxidase method. First, to 100 $\mu\ell$ of 10 $\mu$g/m$\ell$ T cell antigen receptor solution in 0.05 M phosphate buffer (pH 7.5) were added 0.1 mCi of $^{125}$I (manufactured and sold by New England Nuclear Inc., England), 10 $\mu\ell$ of 0.2 mg/m$\ell$ lactoperoxidase solution (manufactured and sold by Calbiochem Inc., U.S.A.; dissolved in a phosphate buffer) and 10 $\mu\ell$ of 0.2 mg/m$\ell$ glucose oxidase solution (manufactured and sold by Calbiochem Inc., U.S.A.). Then, 2 u$\ell$ of 1 M glucose was added to the mixture to start a reaction. The reaction was effected at room temperature for 30 min. Subsequently, 15 $\mu\ell$ of 0.1 M sodium nitride was added to the reaction mixture to stop the reaction. The mixture was dialyzed successively against 0.05 M phosphate buffer containing 0.01 % sodium nitride and against 0.05 M phosphate buffer to obtain $^{125}$I-labeled T cell antigen receptor solution. The thus obtained solution was stored at -20 °C (0.2 m$\ell$; specific activity, 100,000 cpm/$\mu$g).

15 $\mu\ell$ (6000 cpm) of the thus prepared $^{125}$I-labeled T cell antigen receptor solution was mixed with 0.2 m$\ell$ of an RPMI 1640 medium containing 5 v/v% fetal bovine serum and various tumor cell lines (MKN-1, KATO-III, PC-10 and NBT-2) which tumor cell lines were recognized by the clone 51 suspended in the serum (1 x $10^7$ cells/m$\ell$), and kept at 0 °C for 1 hour. Then, the mixture was washed with Hank's solution and subjected to the measurement of the radioactivity. As a result, it was found that the radioactivities of MKN-1, KATO-III, PC-10 and NBT-2 were 3800 cpm, 3600 cpm, 2900 cpm and 3900 cpm, respectively. This result shows that the tumor cell antigen on each tumor cell membrane can be determined by the marker-bonded T cell antigen receptor.

Example 6

A cell-free tumor antigen was determined by T cell antigen receptor as follows.

(Separation of membrane protein from tumor cell NBT-2)

The tumor cell line NBT-2 which was recognized by the clone 51 was cultured in an amount of $1 \times 10^{10}$ cells. The culture was subjected to treatment in substantially the same manner as in Example 1 so that the membrane proteins of the cultured cells were dissolved in the culture medium. The supernatant of the culture was obtained by centrifugation and subjected to dialysis against 10 mM phosphate buffer (pH 7.2, 0.85 % NaCl) to obtain 10 mℓ of a soluble membrane protein fraction.

(Preparation of antiserum against NBT-2 tumor cell membrane protein)

1 mℓ of the above-obtained NBT-2 membrane protein fraction and 1 mℓ of Freund's complete adjuvant were mixed to prepare an emulsion. The emulsion was injected into a BALB/c mouse three times at intervals of one week to effect immunization of the mouse. 3 days after the last injection, a blood sample was collected from the heart of the mouse and allowed to stand still, so that the sample separated into a clotted substance and an antiserum. The antiserum was collected. From the sample, there was obtained 2 mℓ of an antiserum.

(Purification of antiserum by affinity chromatography)

2 mℓ of the NBT-2 tumor cell membrane protein fraction was reacted with 5 mℓ of CNBr-activated Sepharose (manufactured and sold by Pharmacia Fine Chemicals AB, Sweden) to prepare a membrane protein (antibody)-bonded Sepharose. The reaction was effected as follows.

First, 1 g of CNBr-activated Sepharose 4B was sufficiently washed with 1 mM (200 mℓ) HCl on a glass filter (G3), further washed with 200 mℓ of a coupling buffer (0.1 M Na HCO$_3$ buffer, pH 8.3, 0.5 M NaCl) and dried. Then, the membrane protein fraction was dissolved in the coupling buffer in such an amount that the resulting solution had an antibody concentration of 1 mg/mℓ. To 4 mℓ of the thus obtained solution was added the dried Sepharose and the mixture was reacted at room temperature for 2 hours while gently stirring. 2 hours later, 5 mℓ of 0.2 M glycine (pH 8.0) was added to the reaction mixture and the resulting mixture was reacted at room temperature for 2 hours to block excess active groups in the mixture. The obtained mixture was placed on a glass filter (G3) and washed successively with 200 mℓ of the coupling buffer, 200 mℓ of an acetate buffer (0.1 M sodium acetate, pH 4, 0.5 M NaCl) and 200 mℓ of a phosphate buffer (pH 7.0, 0.15 M NaCl) to obtain membrane protein-bonded Sepharose. The membrane protein-bonded Sepharose was packed in a column to obtain an affinity column.

After washing the affinity column sufficiently with the phosphate buffer, 1 mℓ of the antiserum was applied to a column and 500 mℓ of the phosphate buffer was passed through the column to wash out proteins not adsorbed on the column. Then, 20 mℓ of 0.1 M glycine-hydrochloric acid (pH 3.0) was passed through the column to elute the adsorbed antibody. After the pH of the eluate was adjusted to 7, the eluate was concentrated and dialyzed against the phosphate buffer to obtain 1 mg/mℓ of a purified antibody solution.

(Preparation of radioimmunoassay plate)

The antibody solution was diluted 100-fold with the phosphate buffer to a concentration of 10 $\mu$g/mℓ, and added to an assay plate for radioimmunoassay (Falcon 3911, manufactured and sold by Falcon, U.S.A.) in an amount of 100 $\mu$ℓ for each well of the plate. The antibody solution was kept at 4 °C for 48 hours so that the antibody against the NBT-2 membrane protein adheres to the bottom of the plate. The assay plate was then washed sufficiently with a blocking agent (a phosphate buffer containing 3 % BSA) and stored at - 20 °C.

(Radioimmunoassay of tumor cell antigen by $^{125}$I-labeled T cell antigen receptor)

The NBT-2 tumor cell membrane protein fraction was diluted with the blocking agent to prepare a series of solutions having various concentrations. These solutions were separately added to the assay plate having anti-NBT-2 tumor cell protein antibody adhering thereto in an amount of 100 $\mu$ℓ for each well. The solutions in the plate were kept at 37 °C for 2 hours and washed sufficiently with the blocking agent. Then, the clone 51-derived $^{125}$I-labeled T cell antigen receptor solution and the blocking agent were added to the solutions in the wells in amounts of 15 $\mu$ℓ (6000 cpm) and 90 $\mu$ℓ, respectively. The resulting mixtures in the wells were kept at 37 °C for 2 hours. Subsequently, the well was washed sufficiently with the blocking agent, and each well of the plate was cut apart and subjected to measurement of the radioactivity. The results are shown in Table 5.

14

Table 5

| Radioimmunoassay by T cell antigen receptor | | | | | | |
|---|---|---|---|---|---|---|
| Dilution of the solutions (fold) | 10 | 100 | 200 | 1000 | 2000 | 10000 |
| Radioactivity (cpm) | 4530 | 4515 | 4490 | 1115 | 525 | 135 |

The results show that a cell free antigen could be determined by using a labeled T cell antigen receptor.

Example 7

The T cell antigen receptor derived from the clone 51 was obtained in substantially the same manner as in Example 3. To the obtained receptor was bonded a cytotoxin, and its tumor killing activity was evaluated.

(Bonding of a cytotoxin to T cell antigen receptor)

Ricin A chain used as a cytotoxin. The ricin A chain was prepared by a customary method [S. Olsnes, Biochemistry, 12, 3124 (1973)]. The ricin A chain was bonded to the T cell antigen receptor by the method in which N-succinimidyl 3-(2-pyridyldithio) propionate (SPDP) is used [Cancer Res. 42, 5209 (1982)]. Illustratively stated, SPDP was added to 0.5 mℓ of a solution containing the T cell antigen receptor obtained in substantially the same manner as in Example 3. The resulting mixture was kept at 23 °C for 30 min and then the reaction was stopped. Subsequently, the ricin A chain was added to the mixture and a reaction was allowed to proceed at 23 °C for 15 hours to obtain ricin-bonded T cell antigen receptor solution.

(Evaluation of tumor killing activity of cytotoxin-bonded T cell antigen receptor)

10 μℓ of the above-prepared lysin-bonded T cell antigen receptor solution was added to 200 μℓ of an RPMI 1640 medium containing 10 v/v% fetal bovine serum and 2 x $10^5$ cells/mℓ of a cell mixture of the human bladder tumor cell line NBT-2 and the human normal lymphocytes suspended therein. After the mixture was cultured for 24 hours, the mixture was subjected to trypan blue staining to determine life (unstained) or death (stained) of the cells in the mixture. As a result, it was found that only the tumor cells were killed and human normal lymphocytes were not killed. The results showed that the cytotoxin-bonded T cell antigen receptor of the present invention had a tumor killing activity.

**Claims**

1. A cloned T cell, viz. NCACC 85 082 201 and NCACC 85 082 202, which is capable of recognizing plural kinds of tumor cells.

2. An antigen receptor of a T cell, viz. NCACC 85 082 201 and NCACC 85 082 202, which is capable of binding to plural kinds of tumor cells.

3. An antigen receptor according to claim 2, wherein said plural kinds of tumor cells comprise at least two members selected from the group consisting of human tumor cell lines MKN-1, MKN-7, KATO-III, PC-1, PC-9, PC-10, PC-13, PC-14, C-1, M7609, CaR-1, S7512, H-1, HLE, HLF, NBT-2, KU-1, KB, W-2, NRC-12, HBC-4, HBC-6, HeLa, HMV-1, HMV-2, GOTO, SYM-I, YS-K and KYM-1.

4. An antigen receptor according to claim 2 or 3 which is capable of binding to plural kinds of tumor cells and contains a marker bonded thereto.

5. An antigen receptor according to claim 2 or 3 which is capable of binding to plural kinds of tumor cells and contains a cytotoxin bonded thereto.

**Patentansprüche**

1. Eine geklonte T-Zelle, nämlich NCACC 85 082 201 und NCACC 85 082 202, die mehrere Arten von Tumorzellen erkennen kann.

15

**2.** Ein Antigenrezeptor einer T-Zelle, nämlich NCACC 85 082 201 und NCACC 85 082 202, der an verschiedene Arten von Tumorzellen binden kann.

**3.** Ein Antigenrezeptor nach Anspruch 2, wobei die mehreren Arten von Tumorzellen mindestens zwei Linien der folgenden Gruppe von Humantumorzellinien umfassen: MKN-1, MKN-7, KATO-III, PC-1, PC-9, PC-10, PC-13, PC-14, C-1, M7609, CaR-1, S7512, H-1, HLE, HLF, NBT-2, KU-1, KB, W-2, NRC-12, HBC-4, HBC-6, HeLa, HMV-1, HMV-2, GOTO, SYM-I, YS-K und KYM-1.

**4.** Ein Antigenrezeptor nach Anspruch 2 oder 3, der an verschiedene Arten von Tumorzellen binden kann und einen an ihn gebundenen Marker aufweist.

**5.** Ein Antigenrezeptor nach Anspruch 2 oder 3, der an verschiedene Arten von Tumorzellen binden kann und ein an ihn gebundenes Cytotoxin aufweist.

**Revendications**

**1.** Cellule T clonée, à savoir
NCACC 85 082 201 et NCACC 85 082 202, qui est capable de reconnaître plusieurs types de cellules tumorales.

**2.** Récepteur antigénique d'une cellule T, à savoir
NCACC 85 082 201 et NCACC 85 082 202, qui est capable de se lier à plusieurs types de cellules tumorales.

**3.** Récepteur antigénique selon la revendication 2, dans lequel lesdits plusieurs types de cellules tumorales comprennent au moins deux membres du groupe consistant en les lignées cellulaires tumorales humaines MKN-1, MKN-7, KATO-III, PC-1, PC-9, PC-10, PC-13, PC-14, C-1, M7609, CaR-1, S7512, H-1, HLE, HLF, NBT-2, KU-1, KB, W-2, NRC-12, HBC-4, HBC-6, HeLa, HMV-1, HMV-2, GOTO, SYM-I, YS-K et KYM-1.

**4.** Récepteur antigénique selon la revendication 2 ou 3, qui est capable de se lier à plusieurs types de cellules tumorales et auquel est lié un marqueur.

**5.** Récepteur antigénique selon la revendication 2 ou 3, qui est capable de se lier à plusieurs types de cellules tumorales et auquel est liée une cytotoxine.